# EUROPÄISCHE PATENTSCHRIFT

(11) **EP 1 197 563 B1**
(45) Veröffentlichungstag und Bekanntmachung des Hinweises auf die Patenterteilung: **08.03.2006**
(21) Anmeldenummer: 01124428.2
(22) Anmeldetag: 11.10.2001
(51) Int. Cl.: C12P 41/00, C12P 13/04

(54) **Verfahren zur Herstellung von Aminosäuren**
Method for Producing Amino Acids
PROCEDE POUR LA PRODUCTION DES AMINO ACIDES

(30) Priorität: 11.10.2000 DE 10050123
(43) Veröffentlichungstag der Anmeldung: 17.04.2002
(73) Patentinhaber: Degussa AG, 40474 Düsseldorf (DE)
(72) Erfinder: Bommarius, Andreas, Prof. Dr., Atlanta Georgia 30327 (US); Verseck, Stefan, Dr., 63452 Hanau (DE); Drauz, Karlheinz, Prof., 63579 Freigericht (DE)

(56) Entgegenhaltungen:
- EP-A- 0 304 021
- EP-A- 0 474 965
- EP-A- 1 074 628

## Beschreibung

Die vorliegende Erfindung ist auf ein Verfahren zur Herstellung von optisch angereicherten Aminosäuren und auf deren Verwendung gerichtet. Insbesondere richtet sich das Verfahren zum einen auf die Racemisierung, zum anderen auf das Entschützen von speziellen N-geschützten Aminosäuren, im System Acylase/Racemase zur vollständigen Umsetzung von speziellen N-geschützten racemischen Aminosäuren zu optisch reinen Aminosäuren.

Optisch reine Aminosäuren sind für die chemische Synthese sowie die parenterale Ernährung wichtige Ausgangsstoffe. Zur Herstellung optisch reiner Aminosäuren sind dem Fachmann viele Möglichkeiten bekannt. U.a. bieten sich diesbezüglich enzymatische Verfahren an, da sie zum einen katalytisch arbeiten und zum anderen die Aminosäuren mit sehr hohen Enantiomerenanreicherungen herzustellen gestatten.

Es ist prinzipiell bekannt, acetylierte Aminosäuren mittels Aminosäureacylasen in L-Aminosäuren umzuwandeln, doch war man der Ansicht, daß diese sich eigentlich nur für die Spaltung von N-acetylgeschützten Aminosäuren und Aminen/Alkoholen eigenen (EP1197563; A. S. Bommarius et al., Tetrahedron; Asymmetry, 1997, Vol. 8, 3197-3200).

Um den zurückbleibenden D-Acetylanteil ebenfalls nutzen zu können, sind verschiedene Racemisierungsverfahren entwickelt worden. Aus der DE 199 35 268 ist eine Acetylaminosäureracemase bekannt, welche es zusammen im System Acylase/Acetylaminosäureracemase gestattet, racemisches Acetylmethionin vollständig in L-Methionin umzuwandeln.

Aus Streptomyces atratus Y-53 (Tokuyama et al., Appl. Microbiol. Biotechnol. 1994, 40, 835-840) und Amycolatopis sp. TS-1-60 (Tokuyama et al., Appl. Microbiol. Biotechnol. 1995a, 42, 853-859) sind N-Acetylaminosäureracemasen (AAR) bekannt. Von der TS-1-60 weiß man, daß sie in geringem Ausmaß auch N-Carbamoylaminosäuren racemisieren kann.

Trotzdem besteht ein Bedarf an Verfahren, die es erlauben, auch anders als acetyl- oder carbamoyl-geschützte Aminosäuren zu racemisieren und ggf. durch eine darauf folgende enzymatische Schutzgruppenabspaltung zur Gänze in die optisch angereicherte Aminosäure umzuwandeln.

Aufgabe der vorliegenden Anmeldung war deshalb die Angabe eines Verfahrens zur Herstellung von optisch angereicherten Aminosäuren aus einer racemischen Mischung von Aminosäuren, welche mittels einer Urethan- oder Carbamoylschutzgruppe N-geschützt vorliegen.

Die Aufgabe wird durch ein Verfahren mit den Merkmalen des Anspruchs 1 gelöst. Anspruch 2 ist auf den ersten Aspekt der vorliegenden Reaktion - die Racemisierung - gerichtet, während Anspruch 3 den zweiten Aspekt der betrachteten Reaktion - die Schutzgruppenabspaltung betrifft. Ansprüche 4 und 5 betreffen besondere Ausführungsformen der gegenständlichen Umsetzung. Anspruch 6 ist auf eine Verwendung der mittels dieses Verfahrens hergestellten Aminosäuren gerichtet.

Dadurch, daß man ein Verfahren zur Herstellung von enantiomerenangereicherten Aminosäuren bereitstellt, wobei Verbindungen der Formel (I) 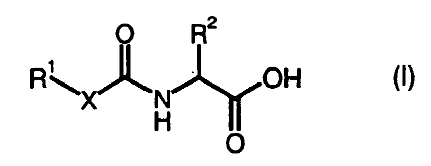
worin
X = O, NH ist,
R¹ = CH₃, CH₃CH₂, tert.-Butyl, Benzyl ist, wobei im Falle von X = NH R¹ = H sein kann,
R² = der α-Rest einer natürlichen oder unnatürlichen Aminosäure ist, mit einem N-Acetylaminosäureracemaseaktivität aufweisenden Enzym (AAR) in Gegenwart oder anschließend mit einem Aminosäureacylaseaktivität aufweisenden Enzym umgesetzt werden, gelangt man völlig überraschend in einer zum Acetylaminosäureweg (DE 199 35 268) äquivalenten dafür aber nicht minder vorteilhaften Art und Weise zu optisch hoch angereicherten Aminosäuren der genannten Art. Es war bisher nicht bekannt, daß Aminosäureacylasen in Verbindung mit Acetylaminosäureracemasen auf oben genannte Verbindungsklasse angewendet werden können.

In einem nächsten Aspekt beschäftigt sich die Erfindung mit einem Verfahren zur Racemisierung von N-geschützten Aminosäuren der allgemeinen Formel (I) 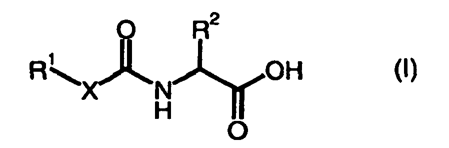
worin
X = O, NH ist,
R¹ = CH₃, CH₃CH₂, tert.-Butyl, Benzyl ist,
R² = der α-Rest einer natürlichen oder unnatürlichen Aminosäure ist,
unter Verwendung eines N-Acetylaminosäureracemaseaktivität aufweisenden Enzyms.

In wiederum einem weiteren Aspekt ist die Erfindung auf ein Verfahren zur Abspaltung der Schutzgruppe von N-geschützten Aminosäuren der allgemeinen Formel (I) 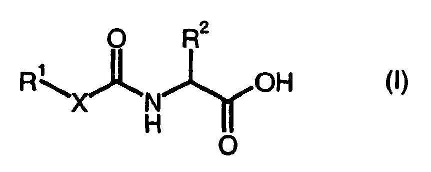
worin.
X = O, NH ist,
R¹ = CH₃, CH₃CH₂, tert.-Butyl, Benzyl ist, wobei im Falle von X = NH R¹ = H sein kann,
R² = der α-Rest einer natürlichen oder unnatürlichen Aminosäure ist,
unter Verwendung eines Aminosäureacylaseaktivität aufweisenden Enzyms gerichtet.

Wie oben schon angedeutet war bisher von beiden Verfahrensaspekten nicht bekannt, daß die entsprechenden eingesetzten Enzyme die genannten Verbindungen erfindungsgemäß umsetzen können. Das Auffinden dieser neuen Aktivität ist mit ursächlich dafür, daß die beschriebenen Enzyme erfolgreich in einem chemischen Prozeß zur Herstellung von Aminosäuren wie eingangs erwähnt eingesetzt werden können.

Unter N-Acetylaminosäureracemase wird eine Klasse von Enzymen bezeichnet, die optisch angereicherte N-Acetylaminosäuren racemisieren kann.
Aufgrund der großen Homologie auf Proteinebene, die diese Enzyme untereinander aufweisen, können im Prinzip alle dem Fachmann bekannten N-Acetylaminosäureracemasen für die vorliegenden Umsetzungen herangezogen werden. Bevorzugt einzusetzende Racemasen sind die aus Streptomyces atratus Y-53 sowie Amycolatopis sp. TS-1-60. Es ist jedoch besonders ein Verfahren bevorzugt, bei dem man die N-Acetylaminosäureracemase aus Amycolatopsis orientalis subspecies lurida (Seq. 2) benutzt, da diese gegenüber den anderen Vertretern dieser Verbindungsklasse Vorteile im Hinblick auf die Metallionenabhängigkeit und Aktivität besitzt (EP 1197563).

Als Aminosäureacylasen werden im Rahmen der Erfindung Enzyme verstanden, welche N-Acylaminosäuren stereospezifisch deacetylieren. Im Prinzip können alle dem Fachmann bekannten Vertreter dieser Verbindungsklasse, die sich für die erfindungsgemäßen Reaktionen eignen, herangezogen werden. Bevorzugt sind allerdings Aminosäureacylasen wie die L-spezifische Acylase I aus Aspergillus oryzae oder eine D-spezifische Acylase. Beide Aminosäureacylasen sind bei der Firma Amano erhältlich. Weitere für die Reaktion einsetzbare Acylasen sind in folgenden Literaturstellen beschrieben: Wakayama M, Yada H, Kanda S, Hayashi S, Yatsuda Y, Sakai K, Moriguchi M, Role of conserved histidine residues in D-aminoacylase from Alcaligenes xylosoxydans subsp. xylosoxydans A-6, Biosci. Biotechnol. Biochem. 2000 Jan;64(1):1-8; Wakayama M, Hayashi S, Yatsuda Y, Katsuno Y, Sakai K, Moriguchi M., Overproduction of D-aminoacylase from Alcaligenes xylosoxydans subsp. xylosoxydans A-6 in Escherichia coli and its purification, Protein Expr. Purif. 1996 Jun;7(4):395-9; Wakayama M, Katsuno Y, Hayashi S, Miyamoto Y, Sakai K, Moriguchi M., Cloning and sequencing of a gene encoding D-aminoacylase from Alcaligenes xylosoxydans subsp. xylosoxydans A-6 and expression of the gene in Escherichia coli, Biosci. Biotechnol. Biochem. 1995 Nov;59(11):2115-9; Wakayama M, Ashika T, Miyamoto Y, Yoshikawa T, Sonoda Y, Sakai K, Moriguchi M.; Primary structure of N-acyl-D-glutamate amidohydrolase from Alcaligenes xylosoxydans subsp. xylosoxydans A-6, J. Biochem. (Tokyo). 1995 Jul;118(1):204-9; Chen HP, Wu SH, Wang KT., D-Aminoacylase from Alcaligenes faecalis possesses novel activities on D-methionine, Bioorg. Med. Chem. 1994 Jan;2(1):1-5; Moriguchi M, Sakai K, Miyamoto Y, Wakayama M., Production, purification, and characterization of D-aminoacylase from Alcaligenes xylosoxydans subsp. xylosoxydans A-6, Biosci. Biotechnol. Biochem. 1993 Jul;57(7):1149-52; Yang YB, Hsiao KM, Li H, Yano H, Tsugita A, Tsai YC, Characterization of D-aminoacylase from Alcaligenes denitrificans DA181, Biosci. Biotechnol. Biochem. 1992 Sep;56(9):1392-5; Tsai YC, Lin CS, Tseng TH, Lee H, Wang YJ, Production and immobilization of D-aminoacylase of Alcaligenes faecalis DA1 for optical resolution of N-acyl-DL-amino acids, Enzyme Microb. Technol. 1992 May;14(5):384-9; Batisse N, Weigel P, Lecocq M, Sakanyan V., Two amino acid amidohydrolase genes encoding L-stereospecific carbamoylase and aminoacylase are organized in a common operon in Bacillus stearothermophilus, Appl. Environ. Microbiol. 1997 Feb;63(2):763-6; Yang YB, Hu HL, Chang MC, Li H, Tsai YC, Purification and characterization of L-aminoacylase from Alcaligenes denitrificans DA181, Biosci. Biotechnol. Biochem. 1994 Jan;58(1):204-5; Jakob M, Miller YE, Rohm KH, Cloning and sequence analyses of cDNAs encoding aminoacylase I from porcine kidney, Biol. Chem. Hoppe Seyler. 1992 Dec;373(12):1227-31; Mitta M, Ohnogi H, Yamamoto A, Kato I, Sakiyama F, Tsunasawa S., The primary structure of porcine aminoacylase 1 deduced from cDNA sequence, J. Biochem. (Tokyo). 1992 Dec;112(6-):737-42; Bommarius AS, Drauz K, Klenk H, Wandrey C., Operational stability of enzymes. Acylase-catalyzed resolution of N-acetyl amino acids to enantiomerically pure L-amino acids, Ann. N Y Acad. Sci. 1992 Nov 30;672:126-36; Gentzen I, Loffler HG, Schneider F., Aminoacylase from Aspergillus oryzae. Comparison with the pig kidney enzyme, Z. Naturforsch. [C]. 1980 Jul-Aug;35(7-8):544-50;

Die erfindungsgemäße Reaktion wird vorzugsweise in einem Enzym-Membran-Reaktor durchgeführt (DE 199 10691).

In einem weiteren Aspekt beschäftigt sich die Erfindung mit der Verwendung der nach Anspruch 1 oder 3 hergestellten Aminosäuren. Im Prinzip können diese in allen dem Fachmann bekannten Nutzungsmöglichkeiten für enantiomer angereicherte Aminosäuren wie parenterale Ernährung oder Tierernährung eingesetzt werden. Vorzugsweise jedoch dienen die optisch angereicherten Aminosäuren zur Synthese bioaktiver Verbindungen.

Die genannten Enzyme können in freier Form als homogen aufgereinigte Verbindungen oder als rekombinant hergestellte Enzyme zusammen oder nacheinander verwendet werden. Weiterhin können die Enzyme auch als Bestandteil eines Gastorganismus (Ganzzellkatalysator wie in (US 6,713,288) eingesetzt werden oder in Verbindung mit der aufgeschlossenen Zellmasse des Wirtsorganismus. Möglich ist ebenfalls die Verwendung der Enzyme in immobilisierter Form (Bhavender P. Sharma, Lorraine F. Bailey and Ralph A. Messing, "Immobilisierte Biomaterialiern - Techniken und Anwendungen", Angew. Chem. 1982, 94, 836-852). Vorteilhafterweise erfolgt die Immobilisierung durch Lyophilisation (Dordick et al. J. Am. Chem. Soc. 194, 116, 5009-5010; Okahata et al. Tetrahedron Lett. 1997, 38, 1971-1974; Adlercreutz et al. Biocatalysis 1992, 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-monocetylether) (Goto et al. Biotechnol. Techniques 1997, 11, 375-378.

Unter optisch angereicherten (enantiomerenangereicherten) Verbindungen wird im Rahmen der Erfindung das Vorliegen einer optischen Antipode im Gemisch mit der anderen in >50 mol-% verstanden.

Unter α-Rest einer Aminosäure wird der am α-C-Atom einer α-Aminosäure befindliche Rest verstanden. Dieser kann sich von einer natürlichen Aminosäure, wie in Beyer-Walter, Lehrbuch der organischen Chemie, S. Hirzel Verlag Stuttgart, 22. Auflage, 1991, S.822f. dargestellt, ableiten. Darüberhinaus sind jedoch auch entsprechende α-Reste unnatürlicher α-Aminosäuren gemeint wie z.B. in DE 199 03 268 aufgeführt.

Der Mikroorganismus Amycolatopsis orientalis subsp. lurida ist unter der Nummer DSM43134 bei der Deutschen Sammlung für Mikroorganismen hinterlegt.

### SEQUENZPROTOKOLL

<110> Degussa-Huels AG
<120> Verfahren zur Herstellung von Aminosäuren
<130> 000399 AM
<140>
   <141>
<160> 2
<170> PatentIn Ver. 2.1
<210> 1
   <211> 1107
   <212> DNA
   <213> Amycolatopsis orientalis
<220>
   <221> CDS
   <222> (1)..(1107)
<400> 1.
   gtg aaa ctc agc ggt gtg gaa ctg cgc cgg gtc cgg atg ccg ctc gtg     48
   Val Lys Leu Ser Gly Val Glu Leu Arg Arg Val Arg Met Pro Leu Val
    1               5                  10                  15
   gcc ccg ttc cgg acg tcg ttc ggg acg cag tcc gag cgg gaa ttg ctg     96
   Ala Pro Phe Arg Thr Ser Phe Gly Thr Gln Ser Glu Arg Glu Leu Leu
               20                  25                  30
   ctg gtc cgc gcg gtg acc ccg gcg ggc gag ggc tgg ggc gaa tgt gtc    144
   Leu Val Arg Ala Val Thr Pro Ala Gly Glu Gly Trp Gly Glu Cys Val
           35                  40                  45
   gcg atg gag gcg ccg ctc tac tcg tcg gag tac aac gac gcc gcc gag    192
   Ala Met Glu Ala Pro Leu Tyr Ser Ser Glu Tyr Asn Asp Ala Ala Glu
       50                  55                  60
   cac gtg ctg cgg aac cat ctg atc ccc gca ctg ctg gcg gcc gag gac    240
   His Val Leu Arg Asn His Leu Ile Pro Ala Leu Leu Ala Ala Glu Asp
   65                  70                  75                  80
   gtg acc gcg cac aag gtg acg ccg ttg ctg gcg aag ttc aag ggc cac    288
   Val Thr Ala His Lys Val Thr Pro Leu Leu Ala Lys Phe Lys Gly His
                   85                  90                  95
   cgg atg gcg aag ggc gcg ctg gag atg gcg gtc ctc gac gcc gaa ctc    336
   Arg Met Ala Lys Gly Ala Leu Glu Met Ala Val Leu Asp Ala Glu Leu
               100                 105                 110
   cgc gcg cat gac cgg tcc ttc gcg gcc gag ctg ggg tcc act cgc gac    384
   Arg Ala His Asp Arg Ser Phe Ala Ala Glu Leu Gly Ser Thr Arg Asp
           115                 120                 125
   tcc gtg gcc tgc ggg gtc tcg gtc ggg atc atg gac tcg atc ccg cac    432
   Ser Val Ala Cys Gly Val Ser Val Gly Ile Met Asp Ser Ile Pro His
           130                     135                     140
   ctg ctc gac gtc gtc ggc ggc tac ctc gac gag ggc tac gtc cgg atc     480
   Leu Leu Asp Val Val Gly Gly Tyr Leu Asp Glu Gly Tyr Val Arg Ile
   145                 150                 155                 160
   aag ctg aag atc gag ccc ggc tgg gac gtc gag ccg gtc cgg cag gtg     528
   Lys Leu Lys Ile Glu Pro Gly Trp Asp Val Glu Pro Val Arg Gln Val
                   165                 170                 175
   cgt gag cgc ttc ggt gac gac gtg ctg ctg cag gtc gac gcg aac acc     576
   Arg Glu Arg Phe Gly Asp Asp Val Leu Leu Gln Val Asp Ala Asn Thr
               180                 185                 190
   gcg tac acg ctg ggc gac gcg ccc ctg ctg tcc cgg ctc gac ccg ttc     624
   Ala Tyr Thr Leu Gly Asp Ala Pro Leu Leu Ser Arg Leu Asp Pro Phe
           195                 200                 205
   gac ctg ctg ctg atc gag cag ccg ctc gaa gaa gag gac gtg ctc ggc     672
   Asp Leu Leu Leu Ile Glu Gln Pro Leu Glu Glu Glu Asp Val Leu Gly
       210                 215                 220
   cac gcc gag ctg gcc aag cgg atc cgg acg ccg atc tgc ctc gac gag     720
   His Ala Glu Leu Ala Lys Arg Ile Arg Thr Pro Ile Cys Leu Asp Glu
   225                 230                 235                 240
   tcg atc gtc tcg gcc aag gcc gcc gcg gac gcg atc aag ctc ggc gcc     768
   Ser Ile Val Ser Ala Lys Ala Ala Ala Asp Ala Ile Lys Leu Gly Ala
                   245                 250                 255
   tgc cag atc gtc aac atc aaa ccg ggc cgg gtc ggc gga tac ctc gaa     816
   Cys Gln Ile Val Asn Ile Lys Pro Gly Arg Val Gly Gly Tyr Leu Glu
               260                 265                 270
   gcc cgc cgg gtg cac gac gtc tgc gcg gca cac ggg atc gcg gtg tgg     864
   Ala Arg Arg Val His Asp Val Cys Ala Ala His Gly Ile Ala Val Trp
           275                 280                 285
   tgc ggc ggg atg atc gag acc ggg ctc ggc cgg gcg gcc aac gtc gca     912
   Cys Gly Gly Met Ile Glu Thr Gly Leu Gly Arg Ala Ala Asn Val Ala
       290                 295                 300
   ctg gcc tcg ctg ccc ggc ttc acg ctg ccg ggg gac acc tcg gcg tcc     960
   Leu Ala Ser Leu Pro Gly Phe Thr Leu Pro Gly Asp Thr Ser Ala Ser
   305                 310                 315                 320
   ggc cgg ttc tat cgc acc gac atc acc gag ccg ttc gtg ctg gac gcc    1008
   Gly Arg Phe Tyr Arg Thr Asp Ile Thr Glu Pro Phe Val Leu Asp Ala
                   325                 330                 335
   ggg cat ctg ccg gtg ccg acc ggg ccg ggc ctc ggg gtg act ccg att    1056
   Gly His Leu Pro Val Pro Thr Gly Pro Gly Leu Gly Val Thr Pro Ile
               340                 345                 350
   ccg gat ctt ctg gac gag gtc acc acg gag aaa gcg tgg atc ggt tcg    1104
   Pro Asp Leu Leu Asp Glu Val Thr Thr Glu Lys Ala Trp Ile Gly Ser
           355                 360                 365
   tag                                                                1107
<210> 2
   <211> 368
   <212> PRT
   <213> Amycolatopsis orientalis
<400> 2

### Beispiele:

### 1. Nachweis der Racemaseaktivität des rekombinanten AAR-Enzyms

Das Substratspektrum der N-Acetylaminosäureracemase aus Amycolatopsis orientalis subsp. lurida wurde mit dem unten beschriebenen Enzymassay getestet.

Der Assay setzte sich wie folgt zusammen:

| | |
|---|---|
| Puffer Tris/HCl | 50 mM (pH 8,0) |
| Substrat | 25 mM |
| Cobaltchlorid | 6 mM |
| AAR | ca. 150 µg gereinigtes Protein |
| Endvolumen | 1 ml |

Im Assay wurden enantiomerenreine Aminosäure-Derivate eingesetzt und die Bildung des entsprechenden Racemats im Polarimeter (Perkin-Elmer 241) verfolgt. Die Inkubation erfolgte bei 30°C (heizbare Küvette) für 3 bis 12 Stunden. Die Messungen erfolgten bei einer Wellenlänge von λ = 365 nm.

**Tabelle 1: Auflistung der getesteten Substrate und der entsprechenden spezifischen Aktivität der AAR.**

| **Substrat** | **Spezifische Aktivität** |
|---|---|
| *N*-Methyloxycarbonyl-L-Met | 42 mU/mg |

### 2. D-Met, bzw. L-Met aus Moc-L-Met

### A. L-Met aus Moc-L-Met:

| | |
|---|---|
| Puffer Tris/HCl | 50 mM (pH 8,0) |
| Moc-L-Met | 25 mM |
| Cobaltchlorid | 6 mM |
| L-Acylase | 2,0 U |

| | |
|---|---|
| (Aspergillus oryzae) | |
| Endvolumen | 200 µL |
| Volumenaktivität: | 1,4 U/ml |

### B. D-Met aus Moc-L-Met:

| | |
|---|---|
| Puffer Tris/HCl | 50 mM (pH 8,0) |
| Moc-L-Met 25 mM | |
| Cobaltchlorid | 6 mM |
| D-Acylase | 2,4 U |

| | |
|---|---|
| (AMANO) | |
| AAR 0,4 U | |
| Endvolumen | 200 µL |
| Volumenaktivität: | 0,6 U/ml |

D-Met, bzw. L-Met wurden über HPLC (RP18) nachgewiesen, die Unit-Angaben der Enzyme beziehen sich auf spezifische Aktivität mit N-Ac-L-Met, bzw. N-Ac-D-Met als Substrat.

## Patentansprüche

1. Verfahren zur Herstellung von enantiomerenangereicherten Aminosäuren, wobei Verbindungen der Formel (I) 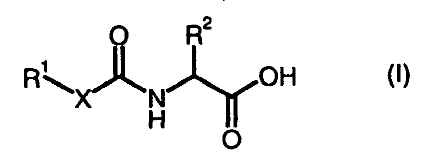
worin
X = O, NH ist,
R¹ = CH₃, CH₃CH₂, tert.-Butyl, Benzyl ist, wobei im Falle von X = NH R¹ = H sein kann,
R² = der α-Rest einer natürlichen oder unnatürlichen Aminosäure ist,
mit einem N-Acetylaminosäureracemaseaktivität aufweisenden Enzym (AAR) in Gegenwart oder anschließend mit einem Aminosäureacylaseaktivität aufweisenden Enzym umgesetzt werden.

2. Verfahren zur Racemisierung von N-geschützten Aminosäuren der allgemeinen Formel (I) 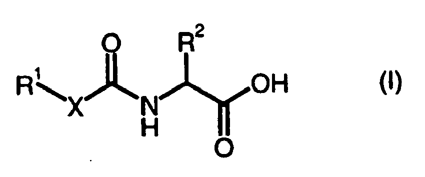
worin
X = O, NH ist,
R¹ = CH₃, CH₃CH₂, tert.-Butyl, Benzyl ist,
R² = der α-Rest einer natürlichen oder unnatürlichen Aminosäure ist,
unter Verwendung eines
N-Acetylaminosäureracemaseaktivität aufweisenden Enzyms.

3. Verfahren zur Abspaltung der Schutzgruppe von N-geschützten Aminosäuren der allgemeinen Formel (I) 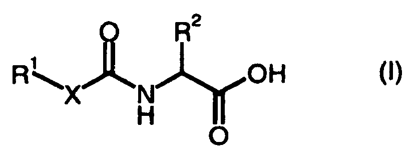
worin
X = O, NH ist,
R¹ = CH₃, CH₃CH₂, tert.-Butyl, Benzyl ist, wobei im Falle von X = NH R¹ = H sein kann,
R² = der α-Rest einer natürlichen oder unnatürlichen Aminosäure ist,
unter Verwendung eines Aminosäureacylaseaktivität aufweisenden Enzyms.

4. Verfahren nach Anspruch 1 oder 2,
**dadurch gekennzeichnet, daß**
man die N-Acetylaminosäureracemase aus Amycolatopsis orientalis subspecies lurida benutzt.

5. Verfahren nach Anspruch 1 oder 3,
**dadurch gekennzeichnet, daß**
man die Acylasen aus Aspergillus oryzae einsetzt.

## Claims

1. Process for preparing enantiomerically-enriched amino acids, wherein compounds of the formula (I) 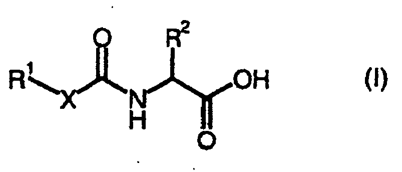
in which
X = O or NH,
R¹ = CH₃, CH₃CH₂, tert-butyl or benzyl, where, when X = NH, R¹ can be H,
R² = the α residue of a natural or unnatural amino acid,
are reacted with an enzyme (AAR) exhibiting N-acetylamino acid racemase activity in the presence of, or subsequently with, an enzyme exhibiting amino acid acylase activity.

2. Process for racemizing N-protected amino acids of the general formula (I) 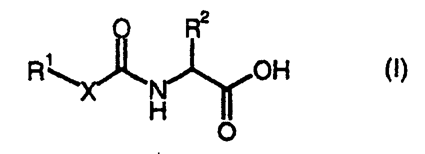
in which
X = O or NH,
R¹ = CH₃, CH₃CH₂, tert-butyl or benzyl,
R² = the α residue of a natural or unnatural amino acid,
using an
enzyme exhibiting N-acetylamino acid racemase activity.

3. Process for eliminating the protective group from N-protected amino acids of the general formula (I) 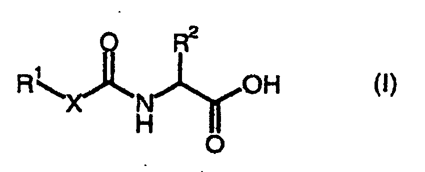
in which
X = O or NH,
R¹ = CH₃, CH₃CH₂, tert-butyl or benzyl, where, when X = NH, R¹ can be H,
R² = the α residue of a natural or unnatural amino acid,
using an enzyme exhibiting amino acid acylase activity.

4. Process according to Claim 1 or 2,
**characterized in that**
the N-acetylamino acid racemase from Amycolatopsis orientalis subspecies lurida is used.

5. Process according to Claim 1 or 3,
**characterized in that**
the acylases from Aspergillus oryzae are used.

## Revendications

1. Procédé de préparation d'acides aminés enrichis en énantiomères, dans lequel des composés de la formule (I) : 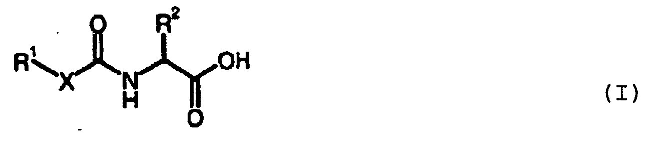
dans laquelle
X = O, NH,
R¹ = CH₃, CH₃CH₂, tert-butyle, benzyle, R¹ pouvant représenter H dans le cas où X = NH,
R² = le radical α d'un acide aminé naturel ou non naturel,
sont mis à réagir avec une enzyme présentant une activité de N-acétylaminoacide-racémase (AAR) en présence de ou par la suite avec une enzyme présentant une activité d'aminoacide-acylase.

2. Procédé de racémisation d'acides aminés protégés en N de la formule générale (I) : 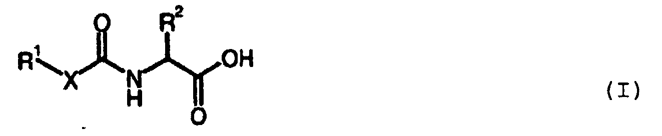
dans laquelle
X = O, NH,
R¹ = CH₃, CH₃CH₂, tert-butyle, benzyle,
R² = le radical α d'un acide aminé naturel ou non naturel,
par utilisation d'une enzyme présentant une activité de N-acétylaminoacide-racémase.

3. Procédé de séparation du groupe de protection d'acides aminés protégés en N de la formule générale (I) : 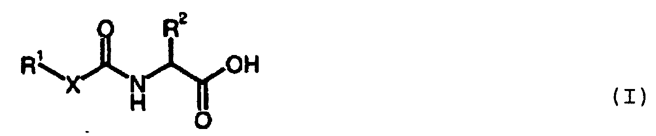
dans laquelle
X = O, NH,
R¹ = CH₃, CH₃CH₂, tert-butyle, benzyle, R¹ pouvant représenter H dans le cas où X = NH,
R² = le radical α d'un acide aminé naturel ou non naturel,
par utilisation d'une enzyme présentant une activité d'aminoacide-acylase.

4. Procédé suivant la revendication 1 ou 2, **caractérisé en ce qu'**on utilise la N-acétylaminoacide-racémase provenant d'Amycolatopsis orientalis subspecies lurida.

5. Procédé suivant la revendication 1 ou 3, **caractérisé en ce qu'**on met en oeuvre les acylases provenant d'Aspergillus oryzae.
